Europäisches Patentamt

European Patent Office

Office européen des brevets

(19)

(11) Numéro de publication: **0 003 693**
**B2**

(12) **NOUVEAU FASCICULE DE BREVET EUROPEEN**

(45) Date de publication du nouveau fascicule du brevet: 21.12.83

(51) Int. Cl.³: **A 61 B 6/02**

(21) Numéro de dépôt: **79400044.8**

(22) Date de dépôt: **23.01.79**

(54) **Appareil de radiographie.**

(30) Priorité: **07.02.78 FR 7803382**

(43) Date de publication de la demande:
**22.08.79 Bulletin 79/17**

(45) Mention de la délivrance du brevet:
**25.02.81 Bulletin 81/8**

(45) Mention de la décision concernant l'opposition:
**21.12.83 Bulletin 83/51**

(84) Etats contractants désignés:
**DE GB NL SE**

(56) Documents cités:
**DE - A - 2 442 009**
**DE - A - 2 655 000**
**FR - A - 2 284 113**
**FR - A - 2 330 057**
**FR - A - 2 330 370**

(73) Titulaire: **THOMSON-CSF**
**173, Boulevard Haussmann**
**F-75379 Paris Cedex 08 (FR)**

(72) Inventeur: **Klausz, Rémy**
**"THOMSON-CSF" - SCPI 173, bld Haussmann**
**F-75379 Paris Cedex 08 (FR)**

(74) Mandataire: **Barbin le Bourhis, Joel et al,**
**THOMSON-CSF SCPI 173, boulevard Haussmann**
**F-75379 Paris Cedex 08 (FR)**

EP 0 003 693 B2

Courier Press, Leamington Spa, England.

## Appareil de radiographie

L'invention concerne un appareil de radiographie et notamment unappareil qui reconstitue une image en traitant par ordinateur les signaux fournis par un détecteur mesurant le coefficient d'absorption d'un corps traversé par un faisceau de rayonnement se déplaçant selon un plan, dit plan de coupe.

Des appareils de ce type, appelés généralement tomodensitomètres, comportent un couple source-détecteur se déplaçant en effectuant un balayage selon le plan de coupe; ces balayages consistent en une succession de translations linéaires effectuées au cours de stations qui sont séparées par des rotations élémentaires dans un plan parallèle au plan de coupe. Les mesures effectuées au cours d'une translation constituent une projection parallèle des coefficients d'absorption selon une direction déterminée, la reconstitution d'une image exigeant un certain nombre de projections également réparties sur un demi-tour.

Dans d'autres appareils connus, pour accélérer les opérations, améliorer l'utilisation du rayonnement émis par la source, et augmenter le nombre total de photons utilisés, on dispose en face de la source plusieurs détecteurs situés dans le plan de coupe, et espacés d'un certain angle formant un éventail dont le sommet est la source. Un tel appareil est décrit par exemple, dans le FR—A 2 337 884, déposée le 7 janvier 1977, au nom de Philips. De nombreux appareils de l'art antérieur utilsent des faisceaux en éventail, tels que par exemple ceux décrits dans les FR—A 2 284 113 de Philips et n° 2 330 370 de Eml Limited ainsi que dans la DE—A 2 655 000 de Eml Limited. On voit qu'avec cette disposition on obtient au cours d'une translation un nombre de projections égal au nombre des détecteurs.

Soit:

$\alpha$   l'angle entre deux détecteurs,
$\Phi$   l'angle de rotation de l'ensemble mécanique portant l'éventail et son système de translation, entre deux stations,
$\theta$   l'angle entre les directions de deux projections successives,
n   le nombre de projections également réparties sur un demi-tour nécessaires pour reconstituer une image correcte,
p   le nombre de détecteurs.

L'angle entre les directions de deux projections successives est donc: $\theta = 180/n$.

La (n+1) ième projection est donc prise avec un angle identique à la première, mais avec un sens inverse de propagation du rayonnement. Le nombre de positions mécaniques, ou stations, nécessaires pour obtenir les n projections dépend des valeurs relatives de $\alpha$, $\theta$ et n. En général on prend $\alpha = \theta$ (l'angle entre deux détecteurs étant égal à l'angle désiré entre deux projections successives) et l'on a alors: $\Phi = p\,\alpha$.

Cet angle est égal à l'angle d'ouverture de l'éventail. On voit donc que l'angle de rotation entre deux stations est d'autant plus élevé qu'on utilise un plus grand nombre de détecteurs. On diminue ainsi le nombre de stations et par conséquent le temps nécessaire à l'obtention des n projections. On utilise généralement cet avantage pour diminuer ce temps et pour améliorer les performances de l'appareil. Pour cela on ne se limite pas à n projections sur un demi-tour, mais on effectue 2 n projections sur un tour complet, ou même davantage. On fait une moyenne des mesures effectuées par les détecteurs à 180 degrés. Ceci permet de remédier, partiellement tout au moins, à divers inconvénients, et notamment à ceux présentés par le non-parallèlisme du faisceau de rayonnement et par le phénomène d'auto-durcissement du faisceau provoqué par l'absorption prioritaire des rayons mous par les masses périphériques du corps à examiner.

Mais d'autres inconvénients subsistent, en particulier ceux provenant des défauts des détecteurs, de leur manque de fiabilité dans le temps, et des différences de réponses d'un détecteur à l'autre pour une même grandeur à mesurer.

Le but de l'invention est de réaliser un appareil de radiographie qui remédie à ces derniers inconvénients.

Certains appareils, tels que ceux décrits dans la demande de brevet français n° 75/08 902 déposée le 21 mars 1975 au nom de EMI Limited, et publiée sous le n° 2 264 516, proposent d'essayer de diminuer les effets de ces inconvénients en adoptant des moyens et des calculs mathématiques relativement complexes.

Selon l'invention on s'arrange d'une part pour que les mesures effectuées à 180° le soient par des détecteurs différents pour chaque point du plan de coupe et, d'autre part, en comparant les mesures effectuées par deux détecteurs voisins, en éliminant les mesures aberrantes, les mesures des détecteurs d'extrémité étant comparées à celles de détecteurs supplémentaires disposés aux extrémités de l'éventail.

L'appareil de radiographie selon l'invention comporte un support pour le corps à examiner, un dispositif monté mobile en translation rectiligne sur un cadre monté mobile en rotation autour du support, le dispositif portant une source émettant un rayonnement en direction de plusieurs détecteurs fixés sur lui, du côté opposé à la source par rapport au support, dans un plan de coupe sensiblement perpendiculaire à l'axe de rotation du cadre. Ces détecteurs sont disposés en éventail par rapport à la source qui en constitue le sommet, et le cadre, mobile en rotation, a plusieurs stations d'immobilisation dans lesquelles le dispositif se déplace en translation, l'angle séparant deux stations voisines étant égal à l'angle d'ouverture de l'éventail, et n'étant pas un sous multiple d'un demi-tour, de telle manière que pour chaque point du plan de coupe, deux mesures effectuées à 180° l'une de l'autre, le soient par des détecteurs différents pour pouvoir comparer les deux mesures et éliminer les mesures aberrantes d'un détecteur defaillant.

Avec cette disposition, les mesures effectuées à 180° en tous points du plan de coupe ne le sont jamais par le même détecteur. En combinant les mesures opposées on peut ainsi remédier, au moins partiellement, à la défaillance d'un détecteur.

Selon une autre caractéristique de l'invention, au moins une extrémité de l'éventail des détecteurs comporte un détecteur supplémentaire.

Les réponses de ces détecteurs supplémentaires sont comparées aux réponses des autres détecteurs et, en cas de défaillance de l'un d'eux, révélée par cette comparaison, il est possible de remplacer ces mesures par celles du détecteur supplémentaire qui les recoupent.

D'autres caractéristiques apparaîtront au cours de la description d'une réalisation donnée ci-après à l'aide des figures qui représentent:

La figure 1, une coupe transversale de l'appareil, et

Les figures 2 et 3, une coupe schématique simplifiée montrant les positions du dispositif mobile dans deux stations.

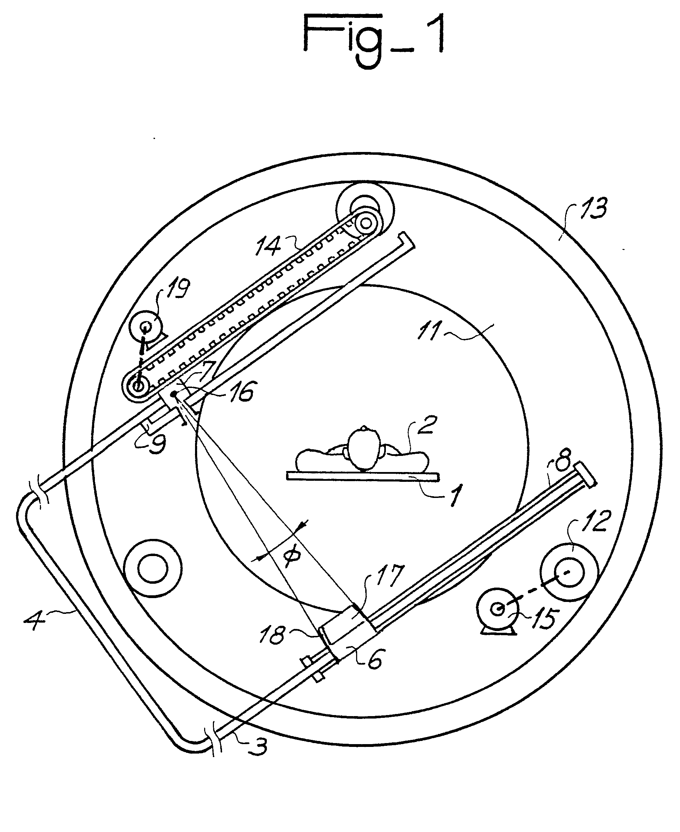

On voit sur la figure 1 un support 1 fixé sur un bâti (non représenté) et sur lequel est placé un corps 2 à examiner. Un dispositif 3 constitué par une pièce 4 en forme de U portant aux extrémités de ses deux branches des patins 6 et 7 coulisse dans des glissières 8 et 9 fixées sur un cadre circulaire 11 roulant par l'intermédiaire de galets 12 mus par un moteur 15 à l'intérieur d'un rail circulaire 13 fixé sur le bâti. Une courroie crantée 14 mue par un moteur 19 est fixée sur le cadre 11 et assure le déplacement des patins 6 et 7 le long des glissières 8 et 9. La source de rayonnement 16 est fixée sur le patin coulissant 7; elle éclaire un bloc de plusieurs détecteurs 17 fixés sur le patin coulissant 6 en émettant un éventail de rayonnement d'ouverture au sommet $\Phi$.

Dans un exemple de réalisation cette ouverture est d'environ 11 degrés 37 minutes, les détecteurs étant au nombre de 30 sans détecteur supplémentaire. Les mesures effectuées par les détecteurs sont envoyées pour traitement à un ordinateur (non représenté).

En fonctionnement, le cadre 11 étant immobilisé dans une position déterminée, le moteur 19 déplace en translation le dispositif 3 par rapport au cadre 11. Pendant ce balayage rectiligne, les détecteurs fournissent les données de p projections, p étant le nombre des détecteurs, les projections étant séparées par un angle $\alpha$.

Après cette translation, le moteur 15 entraîne en rotation le cadre 11 et le dispositif 3 jusqu'à une nouvelle station où un deuxième balayage aura lieu. L'angle $\Phi$ de rotation entre deux stations est pris égal à l'ouverture de l'éventail, c'est-à-dire dans l'exemple numérique précédent 11 degrés 37 minutes.

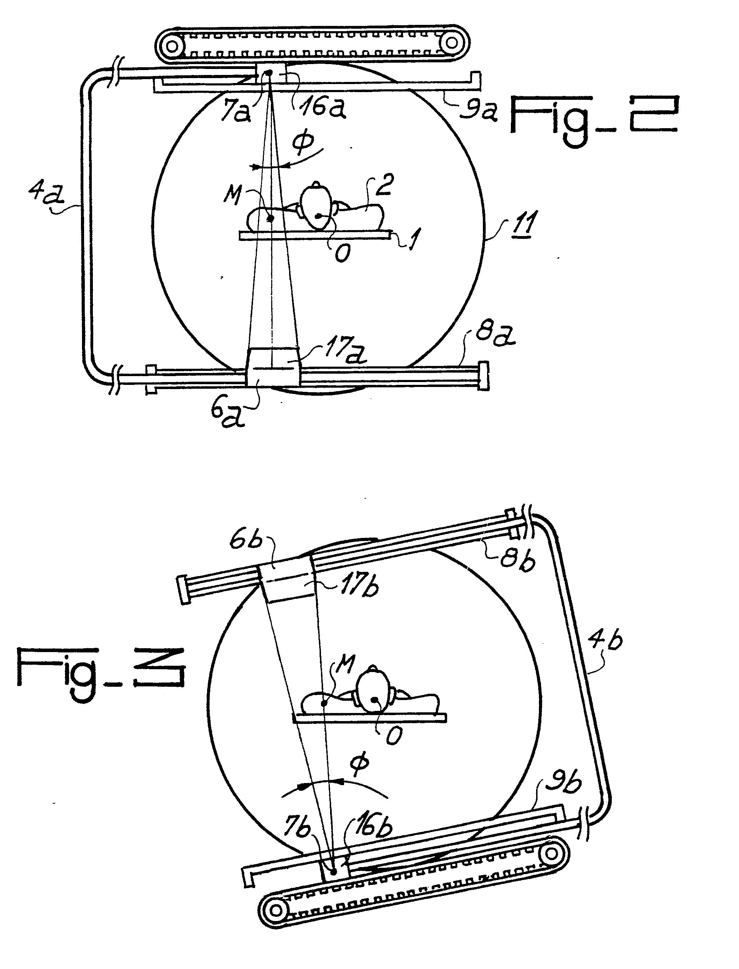

Pour expliciter l'invention on a représenté figures 2 et 3, schématiquement, une coupe de l'appareil avec le dispositif 3 dans deux positions a et b sensiblement opposées. L'indice a (figure 2) ajoute aux repéres correspond à la station d'origine, l'indice b (figure 3) à la station sensiblement opposée. Seule la partie intérieure du cadre 11 a été représentée.

Soit M un point considéré du corps 2 à examiner. Lors d'une première station le point M est traversé par un rayon vertical. Dans le cas de figure le détecteur qui reçoit le faisceau est celui qui est disposé au milieu du bloc 17a des détecteurs. Après un certain nombre de stations espacées angulairement d'un angle égal à $\Phi$, l'éventail se trouve dans la station où le balayage permettra de traverser le point M par un rayon vertical mais en sens inverse du précédent. Cette station porte l'indice b (figure 3).

Selon une caractéristique de l'invention cette station permettant une éclairage inverse du point M ne correspond pas pour le cadre 11 à une position symétrique par rapport à la position initiale.

Autrement dit l'angle $\Phi$ séparant deux stations voisines n'est pas un sous multiple d'un demi-tour. Ceci a pour résultat que le détecteur qui reçoit le rayon vertical en position inverse est différent de celui qui le recevait en position initiale.

Sur les figures 2 et 3 on a représenté le cas où l'angle $\Phi$ est un sous multiple impair d'un tour. Le détecteur concerné dans la position initiale est celui du milieu; en position inverse, le détecteur concerné est celui qui est situé à une extrémité de l'éventail.

On a vu que ce changement de détecteurs pour des mesures de même direction permet d'éliminer ou de remédier à la défaillance d'un détecteur.

Selon une autre caractéristique de l'invention on a ajouté au bloc des détecteurs 17 un détecteur supplémentaire 18 (figure 1). Comme il a été dit plus haut on compare ses réponses à celles des autres détecteurs dans des positions voisines et on élimine les réponses d'un détecteur qui se révèlerait défaillant. Dans ce cas l'angle d'ouverture du faisceau de rayons X doit englober les détecteurs 17 et le détecteur supplémentaire, l'angle de rotation $\Phi$ étant lui toujours égal à l'angle d'ouverture de faisceau de rayons X englobant uniquement les détecteurs 17 sans le détecteur supplémentaire, c'est-à-dire que dans l'exemple numérique précédent, ce détecteur supplémentaire conduit à un nombre total de 31 détecteurs et une ouverture totale de faisceau de 12° (11 degrés 37 minutes + 1/30 de 11 degrés 37 minutes), l'angle rotation $\Phi$ étant toujours égal à 11 degrés 37 minutes.

## Revendications

1. Appareil de radiographie comportant un support (1) pour recevoir un corps (2) à examiner, un dispositif (3) monté mobile en translation rectiligne sur un cadre (11) monté mobile en rotation autour du support (1), ce dispositif (3) portant une source (16) émettant un rayonnement en direction d'un bloc de plusieurs détecteurs (17) fixé sur lui, du côté opposé à la source (16) par rapport au support (1) dans un plan de coupe sensiblement perpendiculaire à l'axe de rotation du cadre (11), ces détecteurs (17) étant disposés en éventail par rapport à la source (16) qui en constitue le sommet, le cadre (11) mobile en rotation ayant plusieurs stations d'immobilisation dans lesquelles le dispositif (3) se déplace en translation, l'angle séparant deux stations voisines étant égale à l'angle (Φ) d'ouverture de l'éventail, appareil caractérisé en ce que l'angle séparant deux stations voisines est autre qu'un sous multiple d'un demi-tour, de telle manière que pour chaque point du plan de coupe deux mesures effectuées à 180° l'une de l'autre, le soient par des détecteurs différents pour pouvoir comparer les deux mesures et éliminer les mesures aberrantes d'un détecteur défaillant.

2. Appareil de radiographie selon la revendication 1, caractérisé en ce qu l'angle séparant deux stations voisines est une fraction impaire d'un tour.

3. Appareil de radiographie selon la revendication 1, caractérisé en ce qu'une extrémité au moins du bloc de plusieurs détecteurs (17) comporte un détecteur supplémentaire, de manière à comparer ses réponses à celles des détecteurs voisins et à éliminer les réponses d'un détecteur défaillant, l'angle séparant deux stations voisines restant égal à l'angle d'ouverture du faisceau englobant uniquement les détecteurs (17) sans le détecteur supplémentaire (18).

## Patentansprüche

1. Röntgenaufnahmegerät mit einem einen zu untersuchenden Körper (2) aufnehmenden Träger (1) und einer auf einem um den Träger (1) drehbeweglich montierten Rahmen (11) geradlinig verschiebbaren Vorrichtung (3), die eine Strahlungsquelle (16) trägt, welche eine Strahlung in Richtung zu einem Block aus mehreren Detektoren (17) aussendet, der an der Vorrichtung auf der der Quelle (16) in bezug auf den Träger (1) in einer im wesentlichen zur Rotationsachse des Rahmens (11) senkrechten Schnittebene gegenüberliegenden Seite befestigt ist, wobei diese Detektoren (17) fächerförmig in bezug auf die Quelle (16) angeordnet sind, die ihren Scheitel bildet, wobei der rotationsbewegliche Rahmen (11) mehrere Haltestationen aufweist, in denen die Vorrichtung (3) eine Translationsverschiebung ausführt, und wobei der zwischen zwei benachbarten Stationen liegende Winkel gleich dem Öffnungswinkel (Φ) des Fächers ist, wobei das Gerät dadurch gekennzeichnet ist, daß der zwischen zwei benachbarten Stationen liegende Winkel keinen ganzzahligen Bruchteil einer Halbdrehung bildet, so daß für jeden Punkt der Schnittebene zwei Messungen, die um 180° gegeneinander versetzt sind, jeweils mit einem anderen Detektor vorgenommen werden, um die zwei Messungen vergleichen zu können und abweichende Messwerte eines fehlerhaften Detektors auszuschalten.

2. Röntgenaufnahmegerät nach Anspruch 1, dadurch gekennzeichnet, daß der zwischen zwei benachbarten Stationen liegende Winkel ein ungeradzahliger Bruchteil einer Umdrehung ist.

3. Röntgenaufnahmegerät nach Anspruch 1, dadurch gekennzeichnet, daß wenigstens ein Ende des mehrere Detektoren (17) aufweisenden Blocks einen zusätzlichen Detektor umfaßt, so daß seine Ansprechsignale mit denen der benachbarten Detektoren verglichen werden und die Antwortsignale eines fehlerhaften Detektors unterdrückt werden, wobei der Winkel zwischen zwei benachbarten Stationen gleich dem Öffnungswinkel des Strahls bleibt, der ausschließlich die Detektoren (17) ohne den zusätzlichen Detektor (18) umfaßt.

## Claims

1. Radiographic apparatus comprising a support (1) adapted to receive a body (2) to be examined, a device (3) movable in a rectilinear translation on a frame (11) rotatably movable about said support (1), said device (3) supporting a source (16) which emits a radiation in the direction of a block comprising a plurality of detectors (17), said block being mounted on said device on the side opposite to said source (16), with reference to said support (1) in a sectional plane substantially perpendicular to the axis of rotation of said frame (11), said detectors (17) being disposed in the manner of a fan with respect to said source (16) which forms the apex thereof, said rotatably movable frame (11) having a plurality of immobilization stations in which said device (3) performs translational movements, the angle defined between two adjacent stations being equal to the apex angle (Φ) of the fan, said apparatus being characterized in that the angle defined between two adjacent stations is different from a submultiple of a semi-revolution in such a manner that for each point of the sectional plane two measurements performed at 180° from each other are taken by different detectors in order to be able to compare the measurements and to eliminate aberrant measurements of a defective detector.

2. Radiographic apparatus according to claim 1, characterized in that the angle defined between two adjacent stations is an odd fraction of one revolution.

3. Radiographic apparatus according to claim 1, characterized in that at least one end of said block comprising a plurality of detectors (17) is provided with a supplementary detector so as to compare the responses of the latter to those of the adjacent detectors and to eliminate the responses of a defective detector, the angle defined between two adjacent stations remaining equal to the opening angle of the beam encompassing only the detectors (17) without the complementary detector (18).

0 003 693

Fig_1

Fig_2

Fig_3